(19) Europäisches Patentamt
European Patent Office
Office européen des brevets

(11) Veröffentlichungsnummer: **0 235 547 B1**

# (12) EUROPÄISCHE PATENTSCHRIFT

(45) Veröffentlichungstag der Patentschrift: **03.04.91**

(51) Int. Cl.⁵: **B01J 23/74**, B01J 23/84, C07C 17/20, C07C 19/08

(21) Anmeldenummer: **87100971.8**

(22) Anmeldetag: **23.01.87**

## (54) Verwendung eines Feststoffkatalysators zur Herstellung von Chlorfluorkohlenwasserstoffen.

(30) Priorität: **01.02.86 DE 3603103**

(43) Veröffentlichungstag der Anmeldung:
**09.09.87 Patentblatt 87/37**

(45) Bekanntmachung des Hinweises auf die Patenterteilung:
**03.04.91 Patentblatt 91/14**

(84) Benannte Vertragsstaaten:
**BE CH DE ES FR GB GR IT LI NL**

(56) Entgegenhaltungen:
**DD-A- 154 603**
**FR-A- 1 082 875**
**FR-A- 1 321 531**
**US-A- 2 946 828**
**US-A- 2 950 258**

(73) Patentinhaber: **HOECHST AKTIENGESELLSCHAFT**
**Postfach 80 03 20**
**W-6230 Frankfurt am Main 80(DE)**

(72) Erfinder: **Müller, Thomas, Dr.**
**Aloifstrasse 17b**
**W-6380 Bad Homburg(DE)**
Erfinder: **Siegemund, Günther, Dr.**
**Frankfurter Strasse 21**
**W-6238 Hofheim am Taunus(DE)**

**EP 0 235 547 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft die Verwendung eines Feststoffkatalysators zur Fluorierung von Chlorkohlenwasserstoffen mit Fluorwasserstoff, vorzugsweise in der Gasphase.

Es ist bekannt, daß Fluorchlormethane sich durch Umsetzung von Chlormethanen mit HF an Antimonhalogenidkatalysatoren in homogener Phase herstellen lassen (Ullmann, Encyklopädie d. techn. Chemie, 4. Aufl., Band 11, S. 636). Dieses Verfahren hat den Nachteil, daß die eingesetzten Rohstoffe wegen der leichten Hydrolysierbarkeit der Antimonhalogenidkatalysatoren wasserfrei sein müssen. Ebenso ist es von Nachteil, daß die Katalysatoren flüchtig sind und unter Druck gearbeitet werden muß.

Es ist auch bekannt, die Chlorfluormethane durch Reaktion der entsprechenden Chlormethane mit HF heterogenkatalytisch in der Gasphase herzustellen. Allerdings zeigt sich z.B. bei der heterogen katalysierten Gasphasenreaktion von Chloroform mit HF der Nachteil, daß die in der Technik üblicherweise eingesetzten Katalysatoren keine ausreichende Selektivität für die Bildung des teilfluorierten Produkts $CHClF_2$ besitzen, sondern bevorzugt die Bildung von $CHF_3$ katalysieren. (US-Patent 29 46 826).

Der in der DE-A-3323374 beschriebene, chrom und $MgF_2$ enthaltende Katalysator führt ebenfalls zu hohen Anteilen an $CHF_3$. Aus der Literatur sind Fluorierungskatalysatoren bekannt, die sich durch eine ausreichende Fluorierungsaktivität auszeichnen und die Gasphasenfluorierung von Chloroform in Richtung eines gewünschten Produktes, vorzugsweise $CHCl_2F$ oder $CHClF_2$, lenken und die Bildung von $CHF_3$ unterdrücken. (DD-PS 154603). Der Katalysator gemäß DD-A-155545 besteht aus Aktivkohle, die mit anorganischen Stickstoffverbindungen behandelt und mit Eisenverbindungen belegt ist.

Die aktive Komponente dieser Katalysatoren ist $FeCl_3$, das auf einen Träger aus Aktivkohle aufgebracht ist. Von Nachteil ist, daß der Aktivkohleträger einer Vorbehandlung, wie z.B. Aufheizen in Gegenwart von Luft oder Ammoniakgas, durch Tränken mit Ammoniumsalz-Lösungen, oder einer Nachbehandlung mit Ammoniak-Lösung unterzogen werden muß, damit er das erforderliche Aktivitäts-/ Selektivitätsverhalten aufweist.

Ein weiterer Nachteil ist, daß diese $FeCl_3$/Kohle-Katalysatoren das erforderliche Aktivitäts-/ Selektivitätsverhalten erst bei einer Reaktionstemperatur von über 200°C aufweisen. Dies bedeutet, daß an das Konstruktionsmaterial, in dem die Reaktion ausgeführt wird, hohe Anforderungen gestellt werden müssen. So ist zum Beispiel Stahl nicht geeignet, da er bei diesen Temperaturen einer Korrosion durch die Reaktionskomponenten, wie HF, HCl und Chlorfluorkohlenwasserstoff ausgesetzt ist. Außerdem führt die Anwesenheit von metallischem Eisen bei diesen Temperaturen zu verstärkter Dismutation, wodurch die schon geringe Selektivität bezüglich gewünschter Zielprodukte noch mehr gesenkt wird (DDR-PS 203 314, Seite 2, Zeile 1).

Fluorchloralkane mit mittlerem Fluorierungsgrad, sind technisch besonders interessant. So wird z.B. Difluorchlormethan ($CHClF_2$) in großem Umfang als Kältemittel und als Ausgangssubstanz zur Herstellung von Polytetrafluorethylen eingesetzt. Die oben genannten Nachteile der bisher bekannten Fluorierungskatalysatoren fallen daher besonders ins Gewicht.

Es bestand somit ein dringendes Bedürfnis nach Katalysatoren für die Gasphasenfluorierung von Chloralkanen, insbesondere Chlormethanen, speziell von Chloroform in Gegenwart von Fluorwasserstoff, die sich durch eine hohe Fluorierungsaktivität und -selektivität auszeichnen. Die Katalysatoren sollen die Fluorierung von Chloroform in Richtung eines mittleren Fluorierungsgrades, vorzugsweise $CHC1F_2$ lenken und die Entstehung unerwünschter Nebenprodukte, insbesondere $CHF_3$, unterdrücken. Die Katalysatoren sollen ihr Aktivitäts- und Selektivitätsoptimum bei Temperaturen unterhalb 200°C haben, was es gestattet, Stahl als Reaktormaterial zu verwenden.

Es wurde nun gefunden, daß sich ein Feststoffkatalysator enthaltend Magnesium-, Eisen-, sowie Kupfer- und/oder Mangan-Kationen in einem analytischem Verhältnis von $3x10^{-3}$ bis 1,5 Grammatom Eisen und 0,01 bis 0,5 Grammatom Kupfer und/oder Mangan pro Grammatom Magnesium, ferner eine zu diesen Kationen äquivalente Menge von Gegenionen, von denen mindestens 75 % Fluoridionen und der Rest Oxid, Hydroxid und/oder Anionen von bei einer Temperatur unter 200°C flüchtigen Säuren sind, sowie gewünschtenfalls inerte Inhaltsstoffe, zur Katalysierung der Umsetzung von Chloralkanen mit Fluorwasserstoff gut eignet, insbesondere zur Umsetzung von Chlormethanen, wie Chloroform. Dieser Katalysator weist bei Temperaturen unter 200°C eine hohe Aktivität und eine hohe Selektivität für die Bildung von Produkten mittleren Fluorierungsgrades auf. Insbesondere gestattet er z.B. die Herstellung von $HCC1F_2$ aus Chloroform und Fluorwasserstoff in überraschend hohen Ausbeuten unter relativ milden Reaktionsbedingungen.

Der erfindungsgemäß eingesetzte Katalysator enthält Magnesium-, Eisen- sowie Kupfer- und/oder Mangankationen, vorzugsweise in einen analytischen Verhältnis von 0,02 bis 0,34 Grammatom Eisen pro Grammatom Magnesium. Die inerten Inhaltsstoffe können z.B. als Streckmittel oder Träger und Gerüstsubstanzen dienen und je nach ihrer Funktion in Mengen von 5-95 % der Gesamtmasse des Katalysators

anwesend sein.

Der Zusatz von Kupfer und/oder Mangan ist im wesentlichen als eine einheitliche Maßnahme zu betrachten. Prinzipiell kann diese Maßnahme durch Zusatz einer dieser Kationen erreicht werden, es können aber auch Kupfer und Mangan gemeinsam in jedem Verhältnis zugesetzt werden. Es hat sich jedoch als besonders zweckmäßig erwiesen, bei Zusatz beider Kationen ein Cu/Mn-Verhältnis von ca. 1:5 bis 5:1, vorzugsweise 2:1 bis 1:2, anzuwenden.

Der Ordnung halber sei bemerkt, daß bei der Herstellung die in den Katalysator eingearbeiteten Kationen in Form der Metalloxide, -hydroxide oder -salze eingesetzt werden.

In dem Katalysator liegen Kupfer und/oder Mangan wie das Magnesium als zweiwertige Kationen, das Eisen als zweioder, vorzugsweise, als dreiwertiges Kation vor.

Die Anionen der bei Temperaturen unter 200°C flüchtigen Säuren leiten sich von organischen oder vorzugsweise anorganischen Säuren ab. Als Anionen anorganischer, bei Temperaturen unter 200°C flüchtiger Säuren, die als Gegenionen zu den obigen Metallkationen fungieren, enthalten die erfindungsgemäßen Katalysatoren z.B. Halogenide, Sulfit, Nitrat oder Carbonat.

Als Halogenide kommen Chlorid, Bromid und Jodid, vorzugsweise aber Fluorid in Betracht. Weitere günstige Gegenionen sind $NO_3^-$, $CO_3^{2-}$, $OH^-$ und $O^{2-}$.
Beispiele für Anionen organischer, bei Temperaturen unter 200°C flüchtiger Säuren sind Formiat, Acetat, Propionat, Halogenacetat wie Mono-, Di- oder Trichloracetat oder Trifluoracetat.

Die Wirksamkeit des erfindungsgemäß eingesetzten Katalysators beruht auf der oben angegebenen qualitativen und quantitativen Zusammensetzung der Wirksubstanz. Die inerten Zusätze, die vorzugsweise frei sind von Chrom, können den Katalysator an die im Einzelfall gegebenen Verfahrensbedingungen anpassen. In der Wirksubstanz des Katalysators sind die angegebenen essentiellen Metallanteile und die entsprechenden Anionenäquivalente gleichmäßig statistisch verteilt.

Die inerten Inhaltsstoffe können unter bestimmten Verfahrensbedingungen die die Gesamtkinetik der Reaktion mitbestimmenden Vorgänge wie z.B. Diffusions-, Adsorptions-, Desorptionsvorgänge beeinflussen oder sie können die erzielte Reaktionsgeschwindigkeit gewünschtenfalls moderieren, um z.B. Temperaturspitzen zu vermeiden. Für die meisten Einsatzzwecke ist die Verwendung des von inerten Inhaltsstoffen freien Katalysators bevorzugt.

Besonders bevorzugt sind Katalysatoren die pro Grammatom Magnesium 0,033 bis 0,28 Grammatome Eisen enthalten und auch Katalysatoren, die pro Grammatom Magnesium 0,03 bis 0,2 Grammatome Kupfer und/oder Mangan aufweisen.

Ferner sind solche Katalysatoren besonders bevorzugt, bei denen das Grammatomverhältnis von Kupfer und/oder Mangan zu Eisen (0,1 bis 5): 1, insbesondere (0,5 bis 2): 1 beträgt, sowie solche, die frei sind von Chrom.

Die erfindungsgemäß eingesetzten Katalysatoren, insbesondere wenn über 95 % der Anionen Fluorid sind, besitzen ihre volle Aktivität bereits vor dem ersten Einsatz im Gegensatz zu Katalysatoren, deren Anionen überwiegend verschieden sind von Flucrid und ihr Wirkungsmaximum erst in Verlauf des ersten Gebrauchs erreichen.

Der von inerten Begleitstoffen freie Wirkstoff des erfindungsgemäß eingesetzten Katalysators kann nach dem oben gesagten durch die Summenformel I

$$Mg\ Fe_a(Cu,Mn)_bX_c \qquad (I)$$

charakterisiert werden, worin
a = $3.10^{-3}$ bis 1,5, vorzugsweise 0,02 bis 0,34
b = 0,01 bis 0,5, vorzugsweise 0,03 bis 0,2
$X_c$ eine zu den Kationen Mg, Fe, Cu und Mn äquivalente Menge Gegenionen, von denen mindestens 75 % Fluoridionen sind und der Rest aus Oxid, Hydroxid und/oder Anionen, bei Temperaturen unter 200°C flüchtiger Säuren besteht.

Die erste Stufe der Herstellung der erfindungsgemäß eingesetzten Katalysatoren kann im Prinzip einfach durch Mischen von Oxiden oder Hydroxiden des Magnesiums, Eisens und gegebenenfalls Kupfers und/oder Mangans oder von Salzen dieser Metalle mit anorganischen bei Temperaturen unter 200°c flüchtigen Säuren bis zur vollständigen Homogenisierung erfolgen.

Eleganter und vorteilhafter ist es jedoch, dies durch eine doppelte Umsetzung zu bewirken. Hierzu wird Magnesiumoxid, Magnesiumhydroxid, oder Magnesiumcarbonat mit einem wasserlöslichen Eisensalz und gegebenenfalls einem wasserlöslichen Kupfer- und/oder Mangansalz im Molverhältnis Magnesium-:Eisen-: Kupfer- und/oder Mangansalz von

1: ($3.10^{-3}$ bis 1,5) : (0,01 bis 0,5),
in wäßrigem Medium bis zur Einstellung des Reaktionsgleichgewichts bei 5 bis 95°C gründlich durchmischt, das Reaktionsgemisch ggf. mit inerten Zusätzen versetzt, getrocknet, gewünschtenfalls zerkleinert und/oder geformt. Schließlich wird zur Herbeiführung der vollen Anfangsaktivität mit Fluorwasserstoff umgesetzt.

Als Kupfer- und Mangansalze werden Salze der zweiwertigen Metalle eingesetzt. Eisen kann in Form des Eisen-II-salzes, vorzugsweise aber in Form des Eisen-III-salzes eingesetzt werden. Als Eisen- II, Eisen-III, Kupfer- II- und Mangan-II-verbindungen können sowohl wasserfreie als auch, vorteilhafterweise, die hydratisierten Salze eingesetzt werden.

Verwendbar sind z.B. die Chloride, und vorzugsweise die Nitrate des Eisens, Kupfers und Mangans. Das eingesetzte Magnesiumoxid darf nicht geglüht sein, sondern muß noch mit schwach sauren Verbindungen reagieren können. Die Umsetzung kann auch mit einem Eisen-III-salz erfolgen, dem pro Mol mindestens 0,01 Mol eines Kupfer-II-salzes und/oder mindestens 0,01 Mol eines Mangan-II-salzes zugesetzt worden ist.

Die Reaktion erfolgt in nicht homogener Phase und benötigt daher eine gewisse Zeit. In der Regel ist bei guter Durchmischung innerhalb von 1 bis 5 Stunden das Reaktionsgleichgewicht erreicht. Die Reaktion kann durch Temperaturerhöhung z.B. bis ca. 95°C beschleunigt werden. Vorzugsweise arbeitet man zwischen 20 und 50°C insbesondere bei Raumtemperatur. Erniedrigte Temperaturen sind möglich, bringen aber keine Vorteile. Das Reaktionsgemisch bildet, soweit nicht unnötig viel Wasser anwesend ist, eine strukturviskose Masse.

Die angewandte Wassermenge für die Reaktion ist nicht kritisch, die Wassermenge muß jedoch ausreichen, damit die Masse zumindest durch eine Kneter verarbeitbar ist. Je höher die eingesetzte Wassermenge, umso mehr Wasser muß schließlich verdampft werden.

Zur Herstellung der Katalysatoren kann man einmal die Eisen- und Kupfer- und/oder Manganverbindung als wäßrige Lösung zu trockenem Magnesiumoxid (oder -hydroxid) geben und die resultierende Reaktionsmischung zu einer Paste verkneten.

Man kann auch das vorgelegte Magnesiumoxid mit Wasser anteigen, die Metallsalze zugeben und die Reaktionsmischung verkneten. Das Verkneten erfolgt vorteilhafterweise mit Maschinen, die in der Verfahrenstechnik üblicherweise zum Vermischen pastöser Stoffe dienen (z.B. Vertikalkneter oder Duplex-Kneter).

Die bei der Herstellung der Katalysatoren erhaltene, strukturviskose Paste wird ohne Waschen getrocknet. Sie ist direkt oder nach dem Trocknen zur Herstellung von Formkörpern geeignet.

Da bei der Herstellung des Katalysators vorteilhafterweise kein Auswaschvorgang eingeschaltet wird, ist das Atomverhältnis Magnesium-Eisen-Kupfer-Mangan der Einsatzkomponenten dem des fertigen Katalysators gleich.

Die Formkörper können mit den üblichen verfahrenstechnischen Maßnahmen, wie z.B. Pelletisieren, Extrudieren oder Granulieren hergestellt werden.

Nach der Formgebung erfolgt die Trocknung der Kontaktformlinge, die zu mechanisch sehr stabilen Katalysatorkörpern führt. Die Trocknung kann sowohl bei Raumtemperatur als auch bei erhöhter Temperatur erfolgen. Zweckmäßigerweise wird eine Trockungstemperatur von 50° bis 150°C, vorzugsweise 70° bis 120°C gewählt, um die Trocknungszeit zu verkürzen. Die Trocknung kann sowohl unter Normaldruck als auch unter Vakuum erfolgen.

Damit die Katalysatoren von vornherein ihre volle Aktivität haben, werden die getrockneten Formlinge oder Pulver mit überschüssigem Fluorwasserstoff bei Temperaturen von 20° bis 500°C behandelt, bevor sie zum Einsatz kommen. Dabei sollen die in den Katalysatoren vorliegenden Anionen unter 200°C flüchtiger anorganischer Säuren, $OH^-$ und $O^{2-}$ zu mindestens 75 %, insbesondere zu über 95 %, durch das Fluoridanion ersetzt werden.

Die Behandlung mit Fluorwasserstoff erfolgt zweckmäßigerweise bei einer Temperatur, bei der flüchtige Reaktionsprodukte nicht kondensieren. Vorteilhaft sind Temperaturen von 100° bis 400°C, vorzugsweise 120° bis 220°C.

Die Fluorierungszeit kann in weiten Grenzen gewählt werden; bevorzugt sind 0,5 bis 10 Stunden. Um das entstehende Wasser und andere flüchtige Reaktionsprodukte schneller zu entfernen und unerwünschte Temperaturspitzen zu vermeiden, kann man HF durch Inertgas (z.B. $N_2$ oder Luft) verdünnen. Die Behandlung mit Fluorwasserstoff zur Aktivierung des Kontaktmaterials kann zweckmäßigerweise in der gleichen Apparatur erfolgen, in der später der Katalysator zur Fluorierung der Chloralkane, vorzugsweise von Chloroform, eingesetzt wird.

Die Katalysatoren sind geeignet zum Einsatz in Festbett-, Fließbett- und Wirbelschichtreaktoren.

Es war überraschend, daß die Katalysatoren das erforderliche Aktivitäts/Selektivitäts-Verhalten schon bei einer Temperatur von utner 200°C zeigen.

Die folgenden Ausführungsbeispiele veranschaulichen die Herstellung und die Verwendung der Katalysatoren.

Beispiel 1 (Vergleichsbeispiel)

1 Gew.-Teil $Fe(NO_3)_3 \cdot 9 H_2O$ wird in 6,7 Gew.-Teilen Wasser aufgelöst. Diese Lösung wird zu 2,77 Gew.-Teilen Magnesiumoxid gegeben und die dabei entstehende pastöse Masse innig verknetet. Anschließend wird das pastöse Reaktionsprodukt zu Würfelförmlingen granuliert und 16 Stunden bei 100°C getrocknet.

0.33 l (Schüttvolumen) der getrockneten Kontaktkörper (ca. 230 g) werden in einem Rohr aus VA-Stahl mit 5 cm lichter Weite und 100 cm Länge bei 200°C mit 5 Mol Fluorwasserstoff behandelt. Die Dauer der Fluorwasserstoffbehandlung beträgt ca. 3 Stunden. Dabei wird HF mit $N_2$ im Molverhältnis 1:2 verdünnt. Das Atomverhältnis Mg/Fe des erhaltenen Katalysators beträgt 27.8.

Beispiel 2

1 Gew.-Teil $Fe(NO_3)_3 \bullet 9 H_2O$ und 1.11 Gew.-Teile $Cu(NO_3)_2 \bullet 3 H_2O$ werden in 7.8 Gew.-Teilen Wasser aufgelöst und zu 2.78 Gew.-Teilen Magnesiumoxid gegeben. Die Weiterverarbeitung und Fluorwasserstoffbehandlung erfolgt gemäß Beispiel 1. In dem erhaltenen Fluorierungskatalysator beträgt das Atomverhältnis Mg:Fe:Cu = 27.8:1:1.85.

Beispiel 3

1 Gew.-Teil Fe $(NO_3)_3 \bullet 9 H_2O$ und 0.56 Gew.-Teile $Cu(NO_3)_2 \bullet 3 H_2O$ werden in 3.3 Gew.-Teilen Wasser aufgelöst. Diese Lösung wird zu 1.39 Gew.-Teilen MgO gegeben. Die Weiterverarbeitung und Fluorwasserstoffbehandlung erfolgen gemäß Beispiel 1. In den beispielsgemäßen Fluorierungskatalysator beträgt das Atomverhältnis Mg:Fe:Cu = 13.9:1:0.93.

Beispiel 4

1 Gew.-Teil $Fe(NO_3)_3 \bullet 9 H_2O$ und 0.63 Gew.-Teile $Mn(NO_3)_2 \bullet 4 H_2O$ werden in 3.75 Gew.-Teilen Wasser aufgelöst. Diese Lösung wird zu 1.5 Gew.-Teilen MgO gegeben. Die Weiterverarbeitung und Fluorwasserstoffbehandlung erfolgt gemäß Beispiel 1. In den beispielsgemäßen Fluorierungskatalysator beträgt das Atomverhältnis Mg:Fe:Mn = 15:1:1.

Unterläßt man bei diesem Beispiel die Fluorwasserstoffbehandlung, so erhält man einen Katalysator mit dem gleichen Atomverhältnis Mg:Fe:Mn, der jedoch seine volle Aktivität erst beim zweiten Fluorierungseinsatz entfaltet.

Beispiel 5

1 Gew.-Teil $Fe(NO_3)_3 \bullet 9 H_2O$ und 0.62 Gew.-Teile $Mn(NO_3)_2 \bullet 4 H_2O$ werden in 1.79 Gew.-Teilen Wasser gelöst. Die Lösung wird zu 0.75 Gew.-Teilen Magnesiumoxid gegeben. Die Weiterverarbeitung und Fluorwasserstoffbehandlung erfolgt gemäß Beispiel 1. In dem beispielsgemäßen Fluorierungskatalysator beträgt das Atomverhältnis Mg:Fe:Mn = 7.5:1:1.

Beispiel 6

1 Gew.-Teil $Fe(NO_3)_3 \bullet 9 H_2O$ und 1.26 Gew.-Teile $Mn(NO_3)_2 \bullet 4 H_2O$ werden in 3.75 Gew.-Teilen Wasser aufgelöst. Die Lösung wird zu 1.5 Gew.-Teilen MgO gegeben. Weiterverarbeitung und Fluorwasserstoffbehandlung erfolgt gemäß Beispiel 1. In dem beispielsgemäßen Katalysator beträgt das Atomverhältnis Mg:Fe:Mn = 15:1:2.

Beispiel 7

1 Gew.-Teil $Fe(NO_3)_3 \cdot 9\ H_2O$ und 0.59 Gew.-Teile $Cu(NO_3)_2 \cdot 3\ H_2O$ und 0.61 Gew.-Teile $Mn(NO_3)_2 \cdot 4\ H_2O$ werden in 5 Gew.-Teilen Wasser aufgelöst. Die Lösung wird zu 1.48 Gew.-Teilen MgO gegeben. Die Weiterverarbeitung und Fluorwasserstoffbehandlung erfolgt gemäß Beispiel 1. In dem beispielsgemäßen Katalysator beträgt das Atomverhältnis Mg:Fe:Cu:Mn = 14.8:1:1:1.

Beispiel 8

Fluorierung von Chloroform in Gegenwart der erfindungsgemäßen Katalysatoren.

438 g Chloroform und 120 g Fluorwasserstoff werden innerhalb von 3 Stunden in gasförmigen Zustand über jeweils 0.33 l des nach Beispiel 1-7 hergestellten Fluorierungskatalysators geleitet. Der Reaktor ist von außen elektrisch beheizbar und besteht aus dem gleichen Rohr, das schon zur Fluorwasserstoffbehandlung bei der Herstellung des Katalysators eingesetzt wurde.

Das Reaktionsgemisch wird nach Verlassen des Reaktors von Fluorwasserstoff und Chlorwasserstoff freigewaschen und gaschromatographisch analysiert. Die Zusammensetzungen der Reaktionsgase sind in Tabelle 1 zusammengestellt.

Tabelle 1 Fluorierungsergebnisse

| Katalysator gem. Beispiel | T°C Reaktor | Reaktionsgaszusammensetzung Vol.% $CHCl_3$ | $CHCl_2F$ | $CHClF_2$ | $CHF_3$ |
|---|---|---|---|---|---|
| 1 | 160 | 19.9 | 30.4 | 39.4 | 8.5 |
| 2 | 160 | 21.8 | 33.7 | 38.1 | 5.3 |
| 3 | 160 | 21.4 | 26.0 | 41.2 | 5.2 |
| 4 | 180 | 5.4 | 24.7 | 64.3 | 5.4 |
| 5 | 180 | 11.4 | 21.9 | 60.9 | 5.8 |
| 6 | 180 | 11.3 | 29.5 | 56.9 | 3.0 |
| 7 | 180 | 11.8 | 19.6 | 62.5 | 5.8 |
| Vergleiche: | | | | | |
| DDR-PS 155 515, S. 4 | 250 | 3.1 | 44.3 | 50.7 | 1.7 |
| " | 270 | 0.4 | 34.6 | 62.5 | 2.5 |
| DE-OS 3 323 374 Beisp. 11 | 207 | 22.3 | 4.7 | 5.6 | 67.4 |

Die Vergleichsangaben zeigen, daß mit bekannten Katalysatoren vergleichbare Produktzusammensetzungen erst bei erheblich über 200° C liegenden Temperaturen zu erhalten sind.

## Ansprüche

1. Verwendung eines Feststoff-Katalysators enthaltend Magnesium-, Eisen-, sowie Kupfer- und/oder ManganKationen in einem analytischem Verhältnis von $3x10^{-3}$ bis 1,5 Grammatom Eisen und 0,01 bis 0,5 Grammatom Kupfer-und/oder Mangan pro Grammatom Magnesium, sowie eine zu diesen Kationen äquivalente Menge von Gegenionen, von denen mindestens 75 % Fluoridionen und der Rest Oxid, Hydroxid und/oder Anionen von bei einer Temperatur unter 200 °C flüchtigen Säuren sind, sowie gewünschtenfalls inerte Inhaltsstoffe, zur Katalysierung der Umsetzung von Chloralkanen mit Fluorwasserstoff.

2. Verwendung gemäß Anspruch 1 zur Katalyse der Umsetzung von Chloroform mit Fluorwasserstoff in der Gasphase.

3. Verfahren zur katalytischen Fluorierung von Chloroform in der Gasphase, wobei man einer Temperatur von 160-250 °C ein Gemisch aus Chloroform und Fluorwasserstoff mit einem Molverhälnis HF/Chloroform von 1,2:1 bis 2.2:1, insbesondere 1,5:1 bis 1,7:1 über einen Feststoff-Katalysator leitet, der Magnesium und Eisen, sowie gewünschtenfalls inerte Inhaltsstoffe enthält, dadurch gekennzeichnet, daß man einen Katalysator verwendet, der Magnesium-, Eisen-, sowie Kupfer- und/oder ManganKationen in einem analytischen Verhältnis von $3x10^{-3}$ bis 1,5 Grammatom Eisen, 0,01 bis 0,5 Grammatom Kupfer und/oder Mangan pro Grammatom Magnesium sowie eine zu diesen Kationen äquivalente Menge an Gegenionen enthält, von denen mindestens 75 % Fluorid und ein etwaiger Rest Oxid Hydroxyd und/oder Anionen von bei Temperaturen unter 200 °C flüchtigen Säuren sind.

4. Verwendung oder Verfahren gemäß Anspruch 1, bzw 3, dadurch gekennzeichnet, daß der Feststoff-Katalysator aus Magnesium-, Eisen-, sowie Kupfer- und/oder ManganKationn, sowie einer zu diesen Kationen äquivalenten Menge von Gegenionen besteht.

## Claims

1. The use of a solid catalyst containing cations of magnesium, iron and copper and/or manganese in an analytical ratio of $3x10^{-3}$ to 1.5 gram atoms of iron and 0.01 to 0.5 gram atom of copper and/or manganese per gram atom of magnesium, and an amount of counter ions equivalent to these cations, of which at least 75% are fluoride ions and the remainder are oxide, hydroxide and/or anions of acids which are volatile at a temperature below 200°C, and if desired inert contents, for catalyzing the reaction of a chloroalkane with hydrogen fluoride.

2. The use as claimed in claim 1, for catalysis of the reaction of chloroform with hydrogen fluoride in the gas phase.

3. A process for the catalytic fluorination of chloroform in the gas phase, in which a mixture of chloroform and hydrogen fluoride having a molar ratio of HF/chloroform of 1.2:1 to 2.2:1, in particular 1.5:1 to 1.7:1, is passed at a temperature of 160-250°C over a solid catalyst containing magnesium and iron and if desired inert contents, which comprises using a catalyst containing cations of magnesium, iron and copper and/or manganese in an analytical ratio of $3x10^{-3}$ to 1.5 gram atoms of iron and 0.01 to 0.5 gram atom of copper and/or manganese per gram atom of magnesium, and an amount of counter-ions equivalent to these cations, of which at least 75% are fluoride and any remainder are oxide, hydroxide and/or anions of acids which are volatile at a temperature below 200°C.

4. The use or the process as claimed in either of claims 1 and 3, wherein the solid catalyst consists of cations of magnesium, iron and copper and/or manganese and an amount of counter- ions equivalent to these cations.

## Revendications

1. Emploi d'un catalyseur solide comprenant des cations magnésium, fer, ainsi que de cuivre et/ou manganèse dans des proportions analytiques de $3x10^{-3}$ à 1,5 atome-gramme de fer et de 0,01 à 0,5

atome-gramme de cuivre et/ou de manganèse bar atome-gramme de magnésium, avec une proportion d'ions complémentaires équivalente à ces cations, parmi lesquels au moins 75% sont des ions fluorure et la partie restante est formée d'ions oxydes ou hydroxydes et/ou d'anions d'acides volatils au-dessous de 200° C, ainsi que si l'on veut des constituants inertes, pour catalyser la réaction de chloroalcanes avec du fluorure d'hydrogène.

**2.** Emploi selon la revendication 1 pour catalyser la réaction du chloroforme avec le fluorure d'hydrogène en phase gazeuse.

**3.** Procédé de fluoration catalytique de cnloroforme en phase gazeuse par passage entre 160 et 250° C d'un mélange de chloroforme et de fluorure d'hydrogène d'un rapport molaire HF/chloroforme compris entre 1,2 et 2,2 en particulier entre 1,5 et 1,7, sur un catalyseur solide contenant du magnésium et du fer avec éventuellement des constituants inertes, procédé caractérisé en ce que l'on utilise un catalyseur contenant des cations de magnésium, fer, ainsi que cuivre et/ou manganèse dans des proportions analytiques de $3x10^{-3}$ à 1,5 atome-gramme de fer et de 0,01 à 0,5 atome-gramme de cuivre et/ou de manganèse par atomegramme de magnésium, avec une proportion d'ions complémentaires équivalente à ces cations, parmi lesquels au moins 75% sont des ions fluorure et la partie éventuellement restante est formée d'anions oxydes ou hydroxydes et/ou d'acides volatils à des températures inférieures à 200° C.

**4.** Emploi ou procédé selon la revendication 1 ou 3, caractérisés en ce que le catalyseur solide est formé de cations de magnésium, fer, ainsi que de cuivre et/ou manganèse, et d'une proportion d'ions complémentaires équivalente à ces cations.